# EUROPEAN PATENT APPLICATION

(11) **EP 4 789 739 A1**
(43) Date of publication of application: **12.08.2026**
(21) Application number: 24882831.1
(22) Date of filing: 23.10.2024
(51) Int. Cl.: A63F 13/67, A63F 13/79, A63F 13/69, A63F 13/80, A63F 13/211, A63F 13/212, A63F 13/25, A63F 13/537, G08B 21/18, A61M 21/00

(54) **APPARATUS AND METHOD FOR PROVIDING ADHD TREATMENT GAME THAT PROVIDES TASKS BASED ON WORKING MEMORY AND ATTENTION**

(30) Priority: 27.10.2023 KR 20230145555; 22.10.2024 KR 20240144533
(71) Applicant: Dragonfly GF Co., Ltd., Seoul 06632 (KR)
(72) Inventor: JO, Chul, Seoul 05698 (KR); LEE, Sejeong, Seoul 08632 (KR); LEE, Hyunwook, Seongnam-si, Gyeonggi-do 13261 (KR); PARK, Jae Young, Bucheon-si, Gyeonggi-do 14602 (KR); NA, Kyoungpil, Seoul 07688 (KR); KANG, Dong Min, Seoul 06369 (KR); OH, Semin, Seoul 06003 (KR); PARK, Young Ju, Seoul 08255 (KR)
(74) Representative: Studio Torta S.p.A.
(86) International application number: PCT/KR2024/016216
(87) International publication number: WO 2025/089804

(57) **Abstract**

The present disclosure relates to an apparatus and a method for providing an attention deficit hyperactivity disorder (ADHD) treatment game, wherein game content focusing on attention and executive functions is provided, and, in particular, tasks based on working memory and attention are provided by adjusting the difficulty, considering working memory. Attention training for reducing omissions due to inattention in users with ADHD, and suppression training for suppressing false alarms (commissions) due to impulsivity in users may be performed repeatedly, thereby providing an improved treatment effect.

## Description

### [TECHNICAL FIELD]

Embodiments of the present disclosure described herein relate to an apparatus and a method for providing games, and more particularly, relate to an apparatus and a method for providing games for ADHD treatment that provide tasks based on a working memory and attention.

### [BACKGROUND ART]

People with Attention Deficit Hyperactivity Disorder (ADHD) experience difficulties in daily life related to attention, executive function, and fine coordination, thereby requiring repetitive educational and therapeutic activities.

In this context, efforts are underway to improve symptoms in people with ADHD by providing them with gamification-based game content designed to enhance attention and executive function, and encouraging them to engage with it.

In conventional games, game content is typically provided in a manner where the difficulty level increases as a player progresses through stages. In particular, when a user's growth or control ability improves through game play, the difficulty level increases to reflect this, but it does not decrease.

On the other hand, to provide therapeutic game content for ADHD users, simply increasing the difficulty level according to stage progression results in game play that does not match the user's condition, making it impossible to perform effective treatment.

Furthermore, in the case of general games, game content is structured around visual-motor coordination, which involves perceiving objects visually and performing actions based on the perceived results, to maintain interest in the game. However, in the treatment of ADHD, it is more important to strengthen training for attention and executive functions than for visual-motor coordination. To improve attention and executive functions, game tasks focused on these specific abilities are required.

In prior patent documents, game outcome data was obtained based on the test subject's performance of a selected game, and WISC-V scores were predicted from this data to determine whether further testing for ADHD was required.

The prior patent documents could only identify the condition of ADHD patients. It was difficult to determine attention deficits or executive function decline tailored to the patient's condition and to provide treatment focused on improving attention and executive functions.

### [DETAILED DESCRIPTION OF THE INVENTION]

### [TECHNICAL PROBLEM]

Embodiments of the present disclosure provide an apparatus and a method for providing a game for treating ADHD that provides game content focused on attention and executive functions, and in particular, provides tasks based on a working memory and attention by adjusting the difficulty level considering the working memory.

Problems to be solved by the present disclosure are not limited to the problems mentioned above, and other problems not mentioned will be clearly understood by those skilled in the art from the following description.

### [TECHNICAL SOLUTION]

According to an embodiment, an ADHD treatment game providing apparatus providing tasks based on a working memory and attention includes a first collection unit connected to a terminal providing game content and acquiring in-game data during gameplay of a user, a second collection unit connected to the terminal and acquiring motion data of a user during gameplay, and a processor that adjusts a weight of a visual coordination task and a weight of an attention training task, which constitute the game content. The attention training task is a game task that determines a monster that a user must avoid during a game, and a monster that a user must defeat during a game, depending on a guide provided before a game. The visual coordination task is a game task that moves the terminal depending on an item that a user must avoid, or an item that a user must acquire. The processor is configured to adjust the weight of the visual coordination task constituting game content within a single stage to be lower than the weight of the attention training task.

According to an embodiment, a game providing method of an ADHD treatment game providing apparatus that provides tasks based on a working memory and attention, which includes a terminal that provides game content, includes acquiring in-game data during gameplay of a user in a state of being connected to a terminal providing game content, acquiring motion data of a user during gameplay in a state of being connected to the terminal, and adjusting a weight of a visual coordination task and a weight of an attention training task, which constitute the game content. The attention training task is a game task that determines a monster that a user must avoid during a game, and a monster that a user must defeat during a game, depending on a guide provided before a game. The visual coordination task is a game task that moves the terminal depending on an item that a user must avoid, or an item that a user must acquire. The adjusting of the weight includes adjusting the weight of the visual coordination task constituting game content within a single stage to be lower than the weight of the attention training task.

Besides, a computer program stored in a computer-readable recording medium for implementing the present disclosure may be further provided.

In addition, a computer-readable recording medium for recording a computer program for implementing the present disclosure may be further provided.

### [ADVANTAGEOUS EFFECTS OF THE INVENTION]

According to the above-described issues solving means of the present disclosure, an enhanced therapeutic effect may be provided by repeatedly performing attention training to reduce omissions caused by inattention of a user with ADHD and inhibition training to suppress commissions caused by impulsivity of the user.

Moreover, according to the above-described issues solving means of the present disclosure, game content capable of training attention and working memory most directly related to ADHD symptoms is provided by adjusting the difficulty level in consideration of attention training tasks rather than visual-motor coordination tasks.

Effects of the present disclosure are not limited to the effects mentioned above, and other effects not mentioned will be clearly understood by those skilled in the art from the following description.

### [DESCRIPTION OF THE DRAWINGS]

FIG. 1 is a block diagram briefly illustrating a configuration of an apparatus for providing games for ADHD treatment that provide tasks based on a working memory and attention, according to an embodiment of the present disclosure.
FIG. 2 is a flowchart illustrating a method of providing a game for an ADHD treatment game providing apparatus that provides tasks based on a working memory and attention, according to an embodiment of the present disclosure.
FIG. 3 illustrates a difficulty adjustment algorithm for an ADHD treatment game providing apparatus that provides tasks based on a working memory and attention, according to an embodiment of the present disclosure.
FIG. 4 illustrates a specific embodiment of placing items or distracting elements for an ADHD treatment game providing apparatus that provides tasks based on a working memory and attention, according to an embodiment of the present disclosure.
FIGS. 5(a) to 6(b) illustrate game content for an ADHD treatment game providing apparatus that provides tasks based on a working memory and attention, according to an embodiment of the present disclosure.
FIG. 7 illustrates a difficulty adjustment algorithm for consecutive failures of an ADHD treatment game providing apparatus that provides tasks based on a working memory and attention, according to an embodiment of the present disclosure.
FIG. 8 illustrates game content for an ADHD treatment game providing apparatus that provides tasks based on a working memory and attention, according to an embodiment of the present disclosure, which performs the acquisition or avoidance of items or monsters.
FIG. 9 illustrates a configuration of a computing device, according to an embodiment of the present disclosure.

### [BEST MODE]

The same reference numerals denote the same elements throughout the present disclosure. The present disclosure does not describe all elements of embodiments. Well-known content or redundant content in which embodiments are the same as one another will be omitted in a technical field to which the present disclosure belongs. A term such as 'unit, module, member, or block' used in the specification may be implemented with software or hardware. According to embodiments, a plurality of 'units, modules, members, or blocks' may be implemented with one component, or a single 'unit, module, member, or block' may include a plurality of components.

Throughout this specification, when it is supposed that a portion is "connected" to another portion, this includes not only a direct connection, but also an indirect connection. The indirect connection includes being connected through a wireless communication network.

Furthermore, when a portion "comprises" a component, it will be understood that it may further include another component, without excluding other components unless specifically stated otherwise.

Throughout this specification, when it is supposed that a member is located on another member "on", this includes not only the case where one member is in contact with another member but also the case where another member is present between two other members.

Terms such as 'first', 'second', and the like are used to distinguish one component from another component, and thus the component is not limited by the terms described above.

Unless there are obvious exceptions in the context, a singular form includes a plural form.

In each step, an identification code is used for convenience of description. The identification code does not describe the order of each step. Unless the context clearly states a specific order, each step may be performed differently from the specified order.

Hereinafter, operating principles and embodiments of the present disclosure will be described with reference to the accompanying drawings.

In this specification, an 'apparatus according to an embodiment of the present disclosure' includes all various devices capable of providing results to a user by performing arithmetic processing. For example, the apparatus according to an embodiment of the present disclosure may include all of a computer, a server device, and a portable terminal, or may be in any one form.

Here, for example, the computer may include a notebook computer, a desktop computer, a laptop computer, a tablet PC, a slate PC, and the like, which are equipped with a web browser.

The server device may be a server that processes information by communicating with an external device and may include an application server, a computing server, a database server, a file server, a game server, a mail server, a proxy server, and a web server.

For example, the portable terminal may be a wireless communication device that guarantees portability and mobility, and may include all kinds of handheld-based wireless communication devices such as a smartphone, a personal communication system (PCS), a global system for mobile communication (GSM), a personal digital cellular (PDC), a personal handyphone system (PHS), a personal digital assistant (PDA), International Mobile Telecommunication (IMT)-2000, a code division multiple access (CDMA)-2000, W-Code Division Multiple Access (W-CDMA), and Wireless Broadband Internet (WiBro) terminal, and a wearable device such as a timepiece, a ring, a bracelet, an anklet, a necklace, glasses, a contact lens, or a head-mounted device (HMD).

Functions related to artificial intelligence according to an embodiment of the present disclosure are operated through a processor and a memory. The processor may consist of one or more processors. In this case, the one or more processors may be a general-purpose processor (e.g., a CPU, an AP, or a digital signal processor (DSP)), a graphics-dedicated processor (e.g., a GPU or a vision processing unit (VPU)), or an artificial intelligence (AI)-dedicated processor (e.g., an NPU). Under control of the one or more processors, input data may be processed depending on an AI model, or a predefined operating rule stored in the memory. Alternatively, when the one or more processors are AI-dedicated processors, the AI-dedicated processor may be designed with a hardware structure specialized for processing a specific AI model.

The predefined operating rule or the artificial intelligence model is created through learning. Here, being created through learning means creating the predefined operating rule or the artificial intelligence model configured to perform desired features (or purposes) as a basic artificial intelligence model is learned by using pieces of learning data by a learning algorithm. This learning may be performed by a device itself, on which the artificial intelligence according to an embodiment of the present disclosure is performed, or may be performed through a separate server and/or system. For example, the learning algorithm may include supervised learning, unsupervised learning, semi-supervised learning, or reinforcement learning, but may not be limited to the above example.

An artificial intelligence model may be composed of a plurality of neural network layers. The plurality of neural network layers respectively have a plurality of weight values, and each of the plurality of neural network layers performs neural network calculation through calculations between the calculation result of the previous layer and the plurality of weight values. The plurality of weight values of the plurality of neural network layers may be optimized by the learning result of the artificial intelligence model. For example, during a learning process, the plurality of weight values may be updated such that a loss value or a cost value obtained from the artificial intelligence model is reduced or minimized. The artificial neural network may include a deep neural network (DNN). The artificial neural network may be, for example, a convolutional neural network (CNN), a deep neural network (DNN), a recurrent neural network (RNN), a restricted Boltzmann machine (RBM), a deep belief network (DBN), a bidirectional recurrent deep neural network (BRDNN), or a deep Q-network, but is not limited to the above-described example.

According to an embodiment of the present disclosure, a processor may implement artificial intelligence. The artificial intelligence may refer to an artificial neural network-based machine learning method that allows a machine to perform training by simulating human biological neurons. The methodology of artificial intelligence may be classified as supervised learning, in which a solution (output data) to a problem (input data) is determined by providing input data and output data together as training data depending on a learning method, unsupervised learning, in which only input data is provided without output data, and thus the solution (output data) to the problem (input data) is not determined, and reinforcement learning, in which a reward is given from an external environment whenever an action is taken in a current state, and thus learning progresses to maximize this reward. Moreover, the methodology of artificial intelligence may also be categorized depending on architecture, which is the structure of the learning model. The architecture of deep learning technology widely used may be categorized into convolutional neural networks (CNN), recurrent neural networks (RNN), transformers, and generative adversarial networks (GAN).

Each of the present device and the system may include an artificial intelligence model. The artificial intelligence model may be a single artificial intelligence model or may be implemented as a plurality of artificial intelligence models. The artificial intelligence model may be composed of neural networks (or artificial neural networks) and may include a statistical learning algorithm that mimics biological neurons in machine learning and cognitive science. The neural network may refer to a model as a whole having the ability to solve problems as artificial neurons (nodes), which form a network by connecting synapses, changes the strength of their synaptic connections through learning. Neurons in the neural network may include the combination of weight values or biases. The neural network may include one or more layers consisting of one or more neurons or nodes. For example, the present device may include an input layer, a hidden layer, and an output layer. The neural network constituting the present device may infer the result (output) to be predicted from an arbitrary input by changing a weight value of a neuron through learning.

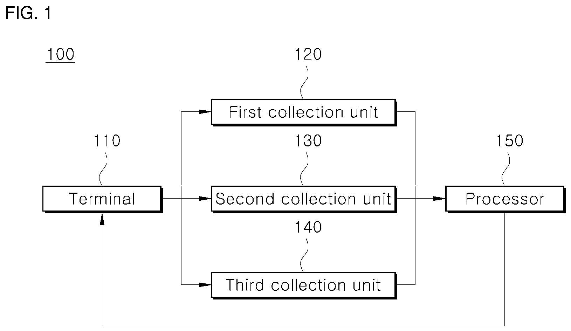

FIG. 1 is a block diagram briefly illustrating a configuration of an apparatus for providing games for ADHD treatment that provide tasks based on a working memory and attention, according to an embodiment of the present disclosure. Hereinafter, with reference to FIGS. 1 to 9, an apparatus for providing games for ADHD treatment that provide tasks based on a working memory and attention according to an embodiment of the present disclosure will be described.

As illustrated in FIG. 1, an ADHD treatment game providing apparatus 100 according to an embodiment of the present disclosure, which provides tasks based on a working memory and attention, may include a terminal 110 that provides game content, a first collection unit 120 connected to the terminal 110 to acquire in-game data during a user's gameplay, and a second collection unit 130 connected to the terminal 110 to acquire motion data of the user playing the game.

Moreover, the ADHD treatment game providing apparatus 100, which provides tasks based on a working memory and attention according to an embodiment of the present disclosure, may include a processor 150 that adjusts the weight of a visual coordination task and the weight of an attention training task constituting the game content,

In an embodiment, the attention training task may be a game task that identifies a monster that the user must avoid during a game, and a monster that the user must defeat during the game, depending on a guide provided before the game.

In an embodiment, the visual coordination task may be a game task that moves the terminal depending on an item that the user must avoid, or an item that the user must acquire. In this case, the movement of the terminal may be identified as being either acquired or avoided through the user's motion data acquired by the second collection unit.

In particular, ADHD may have an attention deficit-dominant type, which is characterized by frequent careless mistakes such as missing details or losing objects and an inability to perform activities systematically, a hyperactivity/impulsivity-dominant type, which is characterized by restlessness, distractibility, impatience, and inability to wait, and a combined type.

In the current classification, the attention deficit-dominant type may exhibit symptoms of inattentive behavior and/or poor executive functions. When the combined type and the attention deficit-dominant type are combined, 80-90% of ADHD patients may exhibit symptoms of inattention and/or poor executive functions.

In particular, for children, it is difficult to distinguish between poor attention and poor executive functions based solely on everyday activities; therefore, for the treatment of ADHD patients, it is necessary to combine training for attention with training for executive function, specifically training for a working memory among the components of the executive function.

In an embodiment, an attention training task may be a game task for performing training on attention and executive functions, and may be game content composed of attention training designed to reduce omissions caused by the user's inattention and inhibition training designed to suppress commissions caused by the user's impulsivity.

At this time, the case where the user does not react to a monster to be defeated may correspond to an omission error, and the case where the user reacts to a monster to be avoided may corresponds to a commission error. Monsters that induce avoidance to suppress unnecessary impulses or behaviors may be non-target stimuli, while monsters that induce treatment to focus on a goal and strengthen attention may be target stimuli.

Non-target stimuli are factors that induce behavioral inhibition; by inducing the user to avoid and ignore monsters, thereby preventing the user from pressing a button. Accordingly, the inhibitory power that is a component of an executive function may be enhanced. On the other hand, target stimuli are factors that induce concentration; by inducing the user to defeat monsters and press the button, thereby enhancing attention.

In an embodiment, the visual coordination task may be game content in which the user performs actions based on the location of an item that must be acquired or avoided to enable visual-motor coordination. This may be a task designed to stimulate interest through quick reflexes, depending on the speed at which items appear, and may serve as game content to complement attention training tasks.

Accordingly, the processor 150 may be configured to adjust the weight of the visual coordination task constituting the game content within a single stage to be lower than the weight of the attention training task. The visual coordination task may be utilized to stimulate user interest such that the training effect is capable of being maintained at a high level while intensively training the attention and the working memory.

In the task described above, the attention training task and the visual coordination task based on visual target stimuli or non-target stimuli are configured as game content. However, in another embodiment, the attention training task and the auditory coordination task based on auditory target stimuli or non-target stimuli may also be configured as game content.

In an embodiment, the terminal 110 may be a device including a touchscreen, and may refer to a device equipped with a touchscreen that allows a user to manipulate at least one object within game content through touch gestures.

The above-described terminal 110 is equipped with communication means for communication with an external server, and may be, for example, a tablet PC or a smartphone equipped with communication functions. The external server may be a server connected to professional medical personnel to perform remote medical treatment based on information collected from the first collection unit 120, the second collection unit 130, and a third collection unit 140. In the meantime, even when remote medical treatment is not performed, professional medical personnel may monitor the status of treatment through a separate website for medical staff and may provide treatment based on the collected information during a hospital visit.

In an embodiment, the first collection unit 120 may be a server connected to the terminal 110 or a storage unit within the terminal 110. The first collection unit 120 may collect in-game achievement rates by collecting information on driving or defeating actions performed by the user within a game.

As an embodiment, the second collection unit 130 may be a gyroscope sensor equipped with a gyroscope function that recognizes motion detection. In the present disclosure, a gyroscope sensor refers to a device equipped with a gyroscope sensor capable of controlling the movement of a character implemented within game content based on left/right directions and the degree of tilt when the user tilts the terminal 110.

For example, it may be a gyroscope sensor embedded in the terminal 110, such as in a tablet PC or smartphone equipped with a gyroscope sensor function, or a separate gyroscope sensor connected to the terminal 110, through which the tilt of the user's terminal 110 or the degree of movement of the terminal 110 is capable of being collected.

In addition, the ADHD treatment game providing apparatus 100, which provides tasks based on a working memory and attention according to an embodiment of the present disclosure, may further include a third collection unit 140 that acquires the user's state data prior to game play.

The third collection unit 140 may be a separate touch screen device or may be the same device as the terminal 110. Before performing the game, a test for inputting a user state may be performed at the terminal 110, or a test for inputting the user state may be performed on a separate touch screen device.

In an embodiment, the user state may be the diagnostic result of a physician who evaluated the ADHD patient before the game content is provided, or may include the answer result of a diagnostic document for evaluating ADHD. Accordingly, the user state, including the degree of decline in the user's attention or executive function, may be identified through the third collection unit 140.

The third collection unit 140 may collect basic information, such as a user ID for identifying data during a treatment process, and user state information related to the execution of the game content, and may identify and process user data.

In an embodiment, the placement of target stimuli or non-target stimuli within the game content may be controlled according to state data input through the third collection unit 140.

For example, when the user has hyperactivity/impulsivity-dominant ADHD, game content that trains an inhibitory function may be provided by placing non-target stimuli after many target stimuli within an attention training task are placed. Alternatively, when the user has attention deficit-dominant ADHD, game content that trains sustained attention may be provided by placing target stimuli after many non-target stimuli are placed.

Alternatively, the user's state data may be obtained according to in-game data acquired from the first collection unit 120 in addition to the state data input through the third collection unit 140.

For example, impairments in the user's attention and executive function may be determined by collecting data regarding the rate or number of successful responses to target stimuli, omission errors (failure to respond to target stimuli), commission errors (responding to non-target stimuli), and successful inhibition (failure to respond to non-target stimuli).

In addition, the game providing apparatus 100 may connect to a separate external server to transmit treatment data, process the data, and provide the processed data to medical professionals.

As an embodiment, the processor 150 may control the first collection unit 120, the second collection unit 130 and the third collection unit 140.

The processor 150 of the game providing apparatus 100 according to an embodiment of the present disclosure is shown in FIG. 1 as a separate block from the terminal 110. However, the processor 150 may be located within a single terminal 110, or may be located on a separate server.

The present disclosure relates to the game providing apparatus 100 for implementing an ADHD digital treatment system for by using gamification. The game content provided by the game providing apparatus 100 may be game-based digital therapeutic content incorporating gamification.

In this case, game content provided such that a patient is incapable of selecting only a game stage or set of a specific difficulty may have a stage or set selected randomly, and the time per stage may also be provided randomly.

Moreover, a display time during which stimulation continues due to non-target stimuli or target stimuli within a game stage may be provided such that it gradually shortens in accordance with the user's success rate.

On the other hand, an inter-stimulus interval-the time interval between the appearance of one non-target or target stimulus and the next-may be randomly varied, regardless of an achievement level at which the user achieves inhibition success or goal success. This variation in an unpredictable manner may serve to heighten the user's attention.

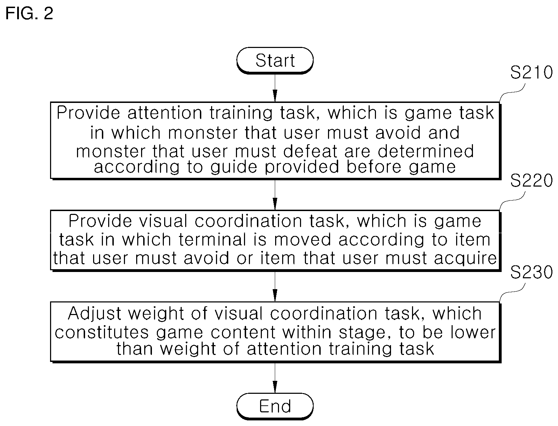

In addition, as illustrated in FIG. 2, a game providing method of the ADHD treatment game providing apparatus 100, which provides tasks based on a working memory and attention according to an embodiment of the present disclosure, may include step S210 of providing an attention training task, which is a game task in which a monster that a user must avoid and a monster that the user must defeat are determined according to a guide provided before a game, step S220 of providing a visual coordination task, which is a game task in which a terminal is moved according to an item that the user must avoid or an item that the user must acquire, and step S230 of adjusting the weight of the visual coordination task, which constitutes game content within one stage, to be lower than the weight of the attention training task.

In an embodiment, the working memory may be referred to as "divided attention".

Prior to step S210, the method may perform a step of acquiring in-game data during the user's gameplay by using the first collection unit 120 and a step of acquiring motion data of the user during the gameplay by using the second collection unit 130.

Before step S210, a step of acquiring the user's state data before the gameplay by using the third collection unit 140 may be further included.

In step S210, an in-game difficulty may be adjusted in real time based on the data acquired from the first collection unit 120, the second collection unit 130, or the third collection unit 140. In this case, real-time adjustment means that weights of target stimuli or non-target stimuli are not adjusted immediately based on the user's success or failure, but adjusted in consideration of consecutive successes or failures.

In the case, the processor 150 according to an embodiment of the present disclosure may provide a guide that provides a monster (a non-target stimulus) that the user must avoid before and during a game, and a monster (a target stimulus) that the user must defeat.

The processor 150 may adjust a difficulty of the game content according to at least one of the number of non-target stimuli or target stimuli, a ratio of the number of the target stimuli to a sum of the number of the non-target stimuli and the number of the target stimuli, a discrimination time limit, and a length of one stage in the attention training task.

For example, the display time may be a time provided to determine whether to tag a target element or to avoid a suppression element, and may be reduced to increase the difficulty of the attention training task.

Alternatively, to increase the difficulty by encouraging the use of more suppression force, the number of target stimuli may be increased, or the number of non-target stimuli may be decreased to increase the ratio of target stimuli.

Alternatively, the length of the entire stage may be increased to raise the difficulty of the attention training task.

Besides, in step S220, the processor 150 may provide an item to be avoided or an item to be acquired, and may allow the terminal 110 to be tilted in the opposite direction of the item to be avoided or to be tilted in the direction of the item to be acquired to acquire or avoid the item. At this time, the item may be in the form of a coin.

In step S230, the weight of the visual coordination task is basically set lower than that of the attention training task, and the difficulty of the visual coordination task may be lowered by the same amount, by which the difficulty of the attention training task is raised by adjusting the difficulty.

In an embodiment, the processor 150 may be configured to output the target element of a guide provided on the prior to a game at the top of the terminal during the game when a performance level of the attention training task of a user is less than a predetermined performance level.

In detail, the user may remember the guide provided before the game and may distinguish between non-target stimuli and target stimuli during the game to decide whether to press a button or not. This increases the capacity of a working memory that the user needs to utilize to perform the attention task.

However, when the performance level is less than a predetermined level (e.g., when the user repeatedly presses a button on a non-target stimulus but not on a target stimulus), target elements may be output on one side of the screen of the terminal 110 providing the game content to assist the user's working memory.

In addition, the processor 150 may be configured to lower the difficulty of the visual coordination task by the same amount, by which the difficulty of the attention training task is increased, to adjust the difficulty of the game content to prevent it from becoming excessive, thereby enhancing the difficulty of the attention training task that affects attention.

In the meantime, in an embodiment, the processor 150 may be configured to additionally provide digit span data at equal or spaced time intervals within the game content in the one stage and to determine whether input user data corresponds to the digit span data.

For example, the terminal 110 may additionally provide a digit span task as game content, in addition to the attention training task and the visual coordination task, may output a number or image unrelated to the game content to the user after the game is performed, and may require the user to immediately input the corresponding number or image.

In an embodiment, when numbers are provided as digit span data, modifications may be applied, such as requiring the user to input the numbers in sequential order, to input the numbers in reverse order, or to change the number of digits. The difficulty may be adjusted to become more difficult as the number of digits increases, such as to 2, 3, or 4 digits.

In an embodiment, the processor 150 may not provide digit span data as game content when the difficulty is low, but may provide digit span data when the difficulty increases.

Alternatively, in another embodiment, the processor 150 may provide digit span data randomly regardless of the stage difficulty, and the difficulty of the provided digit span data may be set according to the user's previous digit span achievement level.

Additionally, the processor 150 may be configured to change the difficulty of the current stage downward after the number of consecutive failures, in consideration of the number of consecutive failures in which the achievement rate of in-game data of the current stage or set did not reach a predetermined value.

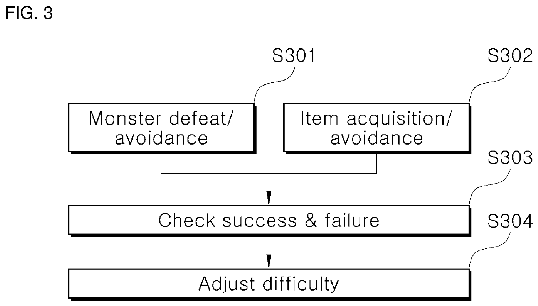

As illustrated in FIG. 3, when a user performs monster defeat/avoidance S301 in step S210 or item acquisition/avoidance S302 in step S220, the processor 150 may check the success or failure of the monster defeat/avoidance S301 or the item acquisition/avoidance S302 based on the user's in-game data (S303).

For example, at the beginning of the game, the frequency of an item to be acquired, an item to be avoided, a monster to be tagged, and a monster to be avoided may be adjusted according to a randomly determined difficulty.

In the middle of the game, after the success or failure of the monster defeat/avoidance S301 or the item acquisition/avoidance S302 is checked a certain number of times or more (S303), difficulty may be adjusted (S304).

When failure is experienced repeatedly, the user's interest or motivation may decrease, which may affect the effectiveness of the treatment. The difficulty may be lowered to focus on attention training tasks by introducing monsters that need to be tagged or avoided, and slowing down the speed at which these monsters disappear, and shortening the stage length.

In this case, the frequency or number of items or monsters may be adjusted to control the difficulty.

For each difficulty level, the number and frequency of items to be acquired, items to be avoided, monsters to be tagged, and monsters to be avoided may be adjusted, and the overall difficulty may be adjusted by varying the number of times that each item or monster appears simultaneously.

In the meantime, the processor 150 may be configured to normally terminate the stage at a random time point within a predetermined game time range, to immediately terminate the stage, and to provide a greater reward than normal termination when an in-game termination element is tagged according to a guide provided prior to the game.

Sustained attention may be trained by randomly changing the game end point such that the user does not predict it.

However, since there is no need to sustain the game for a long time when a certain achievement rate for non-target stimuli or target stimuli has already been achieved in the previous attention training task, an exit element, which ends the game when the user tags it, may be introduced to induce the user to end the game immediately.

Instead of immediately ending the game, the interest of patients with ADHD may be further stimulated by providing immediate and visible rewards, such as offering a greater reward than continuing the game.

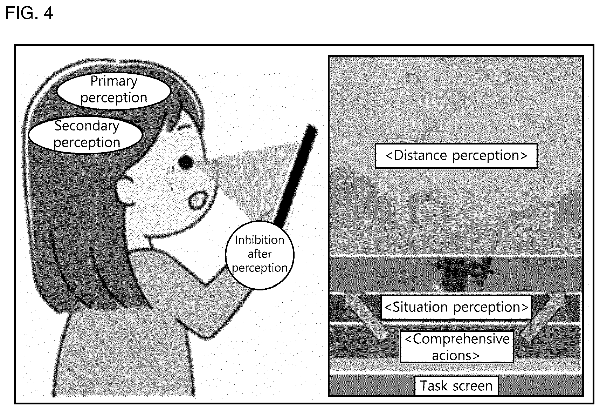

As illustrated in FIGS. 4 to 6, a game providing apparatus according to an embodiment of the present disclosure may determine the location and area of a region where target stimuli and non-target stimuli are to be placed based on the user's primary cognitive ability. An interference region may be established by determining the location and area of a region where non-target stimuli are to be placed.

Then, the number of non-target stimuli relative to the total number of target stimuli and non-target stimuli may be determined such that the user may perform a comprehensive action by determining an avoidance action or a treatment action corresponding to the non-target stimuli based on the user's secondary cognitive ability.

Monsters that serve as target stimuli or non-target stimuli may have the same appearance pattern, but their appearance locations or regions may be set differently depending on the difficulty.

Moreover, the load of a working memory may be determined to determine how many monsters appear and how many of them are non-target stimuli, thereby adjusting the difficulty.

Accordingly, an additional difficulty may be adjusted by adjusting the number or region of non-target stimuli provided in game content to match the user's perceptual range based on the difficulty, or by adjusting the movement direction or speed of a target stimulus or a non-target stimulus.

Referring to FIGS. 4 to 6, the user may perceive distances from non-target stimuli or target stimuli through primary perception, and may perform comprehensive situational awareness such as the movement of the target through secondary perception, thereby performing comprehensive actions by taking avoidance or treatment actions after perception.

Accordingly, the processor 150 of the game providing apparatus 100 according to an embodiment of the present disclosure may also adjust locations, areas, or ratios of non-target stimuli within the game content according to a random difficulty.

The processor 150 according to an embodiment of the present disclosure may expand a region, where non-target stimuli appear, as the difficulty increases, and may place non-target stimuli in the proximity region of the motion manipulation unit of a target within the game content.

For example, in the game content of a low difficulty, non-target stimuli may be limited to only a part of the top region within the game content, and monsters to be avoided may appear only in that part of the top region. On the other hand, in the game content of a high difficulty, non-target stimuli may be expanded to the entire upper region within the game content, or may not be limited to the upper region within the game content, and monsters to be avoided may appear in a wider region.

In the meantime, in addition to visual tasks, the game content may consist of an attention training task and an auditory coordination task based on auditory target stimuli or auditory non-target stimuli.

The processor 150 may be configured to output sounds corresponding to monsters that the user must avoid during a game and monsters that the user must defeat during the game, according to an auditory guide provided before the game, or to visually place and output sounds corresponding to monsters that the user must avoid during the game and monsters that the user must defeat during the game, according to a visual or auditory guide provided before the game.

In other words, the game content may be composed of an attention training task that provides non-target stimuli or target stimuli visually or auditorily.

In an embodiment, the user may remember monsters to be avoided, which are non-target stimuli, or monsters to be defeated, which are target stimuli, based on a visual guide provided prior to the game, and may click on a screen within the terminal 110 during the game according to the non-target stimuli or target stimuli.

In an embodiment, before a game of the terminal 110 is executed, a Monster Guide User Interface (UI) may be displayed on the screen such that the user may perceive or remember target stimuli monsters and non-target stimuli monsters in advance. Afterward, when a monster appears on the screen, the movements of characters may be set to tag monsters being target stimuli or to avoid monsters being non-target stimuli by touching anywhere on the screen.

Alternatively, the user may tilt the screen of the terminal 110 to move in the direction of an item to be acquired or in the direction of no item to be avoided.

Alternatively, in another embodiment, the user may remember the sounds of suppression elements or target elements according to an auditory guide provided before the game, and may click a button or the screen in response to sounds that occur during the game.

In addition, the processor 150 may be configured to display the achievement level for the attention training task as the response rate to the target stimuli, the omission rate to the target stimuli, the commission rate to the non-target stimuli, the non-response rate to the non-target stimuli, the response speed, and the standard deviation of the response speed, and to display the achievement level for the visual coordination task as the correct answer rate based on whether items to be avoided are avoided and whether items to be acquired are acquired.

The attention training task may be an important task for ADHD treatment, and its achievement level may be displayed by using various parameters. On the other hand, the visual coordination task may be an auxiliary task for ADHD treatment, and its achievement level may be displayed by using a single parameter.

For example, with regard to the visual coordination task, only the accuracy rate based on whether an acquisition item was obtained or an avoidance item was avoided may be output. As an auxiliary task related to attention, a simplified success or failure status may be provided as an auxiliary indicator.

With regard to the attention training task, detailed information may be provided by displaying the achievement level by using a plurality of parameters, such as whether an inhibition element is avoided or causes a false alarm, whether a target element is reacted or missing, and the reaction speed or standard deviation of the reaction speed regarding the target element. As a major task related to attention, ability scores such as attention, inhibition, and working memory may be specifically determined from the plurality of parameters.

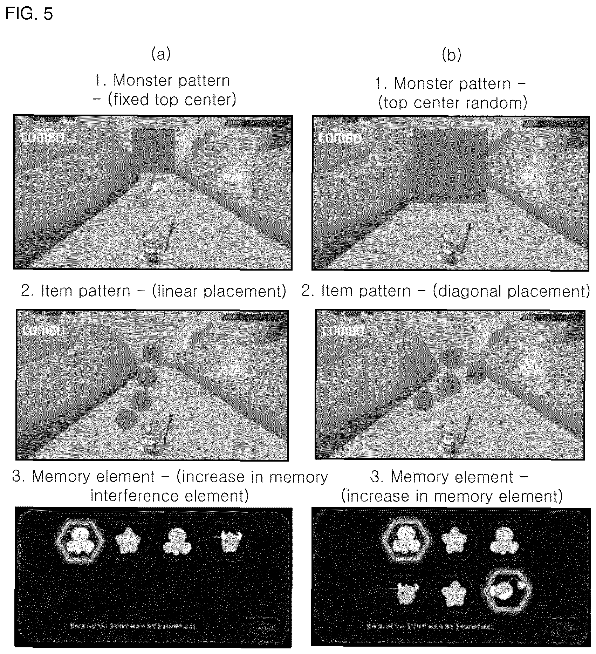

In the meantime, as illustrated in FIGS. 5(a) to 6(b), the processor 150 may determine the difficulty by differently determining a monster appearance pattern, an item appearance pattern, and the number of memory elements. The monster appearance pattern may include patterns in which monsters to be avoided or monsters to be defeated appear, and the item appearance pattern may include patterns in which items to be avoided or items to be acquired appear. A distracting element within the game content may refer to the placement of non-target stimuli including monsters to be avoided or items to be avoided.

The difficulty may be determined by adjusting monster appearance locations while monster appearance patterns are not changed.

As illustrated in FIG. 5(a), the appearance pattern of acquisition monsters or avoidance monsters may be fixed to the top center, and acquisition items or avoidance items may be placed in a straight line.

When the difficulty is increased beyond this, as illustrated in FIG. 5(b), the appearance pattern of acquisition monsters or avoidance monsters may be placed in the center but set randomly, and acquisition items or avoidance items may be placed diagonally.

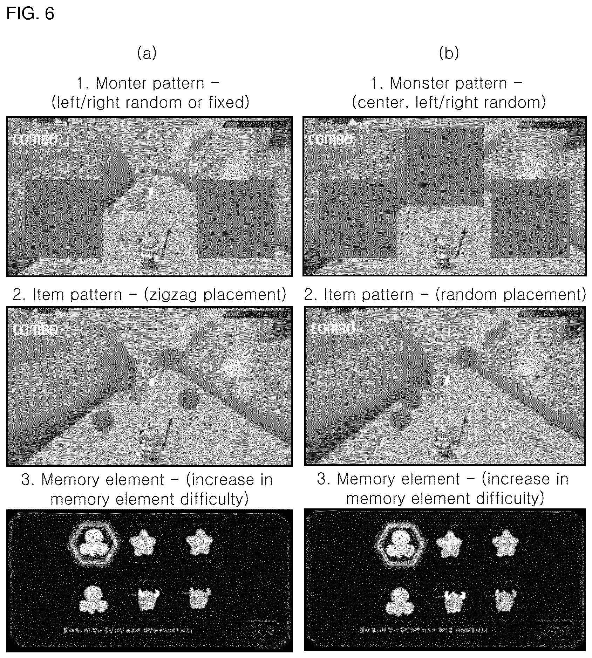

Alternatively, as illustrated in FIG. 6(a), the appearance pattern of acquisition monsters or avoidance monsters may be placed randomly to the left or right, or fixed to the left or right, and acquisition items or avoidance items may be placed in a zigzag pattern.

Alternatively, as illustrated in FIG. 6(b), the appearance pattern of acquisition monsters or avoidance monsters may be placed in the center or randomly to the left or right, and acquisition items or avoidance items may be placed randomly.

In addition, the difficulty may be increased by increasing the number of kill monsters or avoidance monsters, which are memory elements, or by changing their ratio.

Accordingly, the frequency or appearance pattern of the target stimuli or non-target stimuli may be adjusted according to the difficulty of the game content, and the range of increase or decrease in the execution speed of the target stimuli or non-target stimuli may be further adjusted.

This difficulty may be determined randomly. During game progression, the difficulty may be adjusted at predetermined time intervals in consideration of the number of consecutive successes or failures.

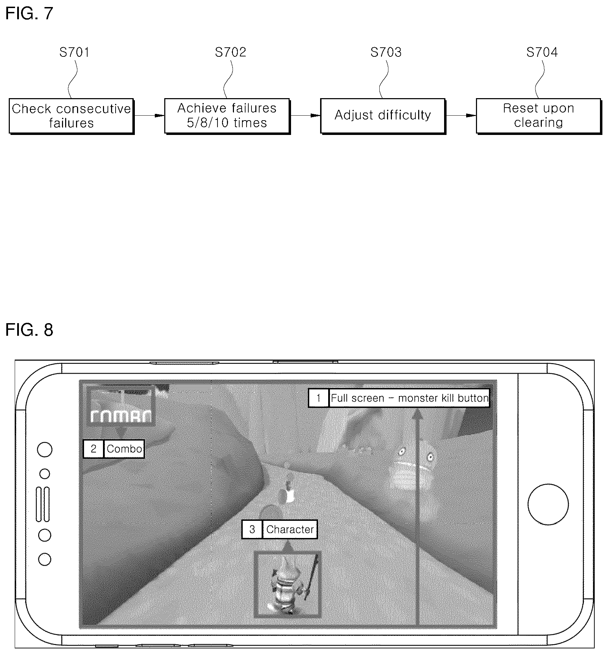

In detail, as illustrated in FIG. 7, the achievement results of items or monsters may be collected through the first collection unit 120, which acquires game data by checking whether an acquisition item has been acquired or an avoidance item has been avoided among items placed according to the game progression order, and checking whether an acquisition monster has been defeated or an avoidance monster has been avoided among monsters placed according to the game progression order.

In other words, the processor 150 may change the difficulty of the current stage downward in consideration of the number of consecutive failures in which the achievement rate of in-game data of the current stage or set did not reach a predetermined value.

As illustrated in FIG. 7, the processor 150 may check consecutive failures of a user based on the results collected from the first collection unit 120 (S701). In the case where the achievement rate performed multiple times in each stage or each set does not reach a predetermined achievement rate, the case may be determined as a failure.

In this case, when the stages or sets determined to be failures are consecutive, the number of consecutive times may be considered. For example, when failures are achieved 5/8/10 times consecutively (S702), the difficulty may be adjusted according to the number of consecutive failures (S703).

In other words, instead of immediately determining success or failure for each attempt to adjust the difficulty, the success or failure may be determined based on the predetermined number of attempts unknown to the user, for example, 5, 8, or 10 attempts. When the accumulated number of successes or failures does not exceed a predetermined number, the difficulty may be lowered.

In the case, to lower the difficulty, at least one of the frequency, ratio, or number of target elements or avoidance elements related to the attention training task may be adjusted.

For example, assuming that the difficulty in S702 is represented only as -2, -1, 0, 1, and 2, the maximum set difficulty may be changed to 1 when there are 5 consecutive failures. When there are 8 consecutive failures, the maximum set difficulty may be maintained at 1. From the condition of 8 consecutive failures, it may be set to restart while the current set difficulty may be lowered to -1. When there are 10 consecutive failures, the current set difficulty may be lowered to 0. From the condition of 10 or more consecutive failures, it may be set to restart while the current set difficulty may be lowered to -2.

Then, when the user succeeds by exceeding a predetermined achievement rate, the existing number of failures may be reset. In this way, the game content may be configured to prevent user deprivation and to maintain focus.

Accordingly, the maximum or minimum value of the real-time difficulty may change depending on the user's in-game data, and the current difficulty therebetween may also change.

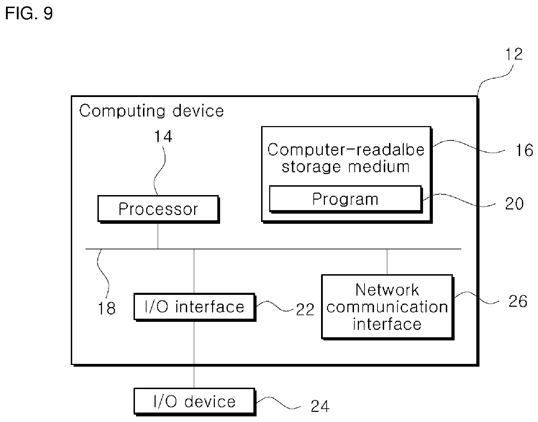

In the meantime, the configuration of game content provided to the terminal 110 may be configured as illustrated in FIGS. 8 and 9.

Referring to FIG. 8, a character may be positioned in the center of the screen in the in-game screen; a stage progress bar may be displayed in the upper right corner; and, a combo achievement rate may be displayed in the upper left corner. This is intended to enhance the therapeutic effect by displaying the combo success rate and encouraging the user to concentrate on achieving higher combos.

Although it is displayed as the combo achievement rate in FIG. 8, as described above, the achievement level for the attention training task may be displayed as a response rate to the target element, an omission rate to the target element, a commission rate to the inhibitory element, a non-response rate to the inhibitory element, and a reaction speed and the standard deviation of the reaction speed, and the achievement level for the visual coordination task may be displayed as the correct answer rate based on whether items to be avoided are avoided and whether items to be acquired are acquired.

Moreover, although the stage progress bar is displayed in FIG. 8, the stage progress bar may be omitted such that a user is capable of maintaining attention until the end of the stage, as the user does not know when the stage will end.

The character's movement may be set to tag monsters being target stimuli or to avoid monsters being non-target stimuli by touching anywhere on a screen, or the character may be set to move in a direction in which a coin to be acquired is located or in a direction where there is no coin to be avoided by tilting the screen of the terminal 110.

Furthermore, the game providing apparatus 100 according to an embodiment of the present disclosure may further include an artificial intelligence model that collects data from the first collection unit 120 or the second collection unit 130 to determine whether the user's state has improved.

The artificial intelligence model may adjust the difficulty of the current stage or the current set according to the degree of state improvement. It may be configured to evaluate the user's game adaptation ability based on user state information and character state information, and to change at least one of play mission information, levels of appearing monsters, and levels of provided items based on the user's game adaptation ability.

Besides, the game providing apparatus 100 according to an embodiment of the present disclosure may further include a device or an auxiliary program (e.g., a site for medical staff, an application for guardians) that is remotely connected to the first collection unit 120, the second collection unit 130, and the third collection unit 140 to display collection results, and an alarm unit that provides an alarm when the amount of change in improvement and deterioration of the collection results is greater than or equal to a moving average value of the amount of change in improvement and deterioration within a preset period.

An external server connected to the first collection unit 120, the second collection unit 130, or the third collection unit 140 may provide data to professional medical staff through the device or the auxiliary program connected to the external server, and may provide information such that a doctor determines whether to perform treatment based on information collected by the first collection unit 120, the second collection unit 130, and the third collection unit 140, such as user data logs and motion data.

In this case, the device or the auxiliary program may organize and provide information collected through a separate processor, such as charting or graphing. When meaningful information is detected from the provided information, the alarm unit may provide an alarm.

Meanwhile, FIG. 9 illustrates a computing device, according to an embodiment of the present disclosure. As shown in FIG. 9, the game providing device 100 according to an embodiment of the present disclosure may be implemented in a form of a recording medium storing computer-executable instructions. The instructions may be stored in a form of program codes, and, when executed by a processor, generate a program module to perform operations of the disclosed embodiments. The recording medium may be implemented as a computer-readable recording medium.

The game providing device 100 according to an embodiment of the present disclosure may correspond to a computing device 12, and the computing device 12 may include at least one processor 14, a computer-readable storage medium 16 including a program 20, and a communication bus 18. In the case, the processor 14 may be the same processor as the processor 150 described above.

Moreover, the computing device 12 may include one or more input/output (I/O) interfaces 22 that provide interfaces for I/O devices 24, and one or more network communication interfaces 26.

Disclosed embodiments are described above with reference to the accompanying drawings. One ordinary skilled in the art to which the present disclosure belongs will understand that the present disclosure may be practiced in forms other than the disclosed embodiments without altering the technical ideas or essential features of the present disclosure. The disclosed embodiments are examples and should not be construed as limited thereto.

## Claims

1. An ADHD treatment game providing apparatus providing tasks based on a working memory and attention, the apparatus comprising:
a first collection unit connected to a terminal providing game content and configured to acquire in-game data during gameplay of a user;
a second collection unit connected to the terminal and configured to acquire motion data of the user during the gameplay; and
a processor configured to adjust a weight of a visual coordination task and a weight of an attention training task, which constitute the game content,
wherein the attention training task is a game task that determines a monster that the user must avoid during a game, and a monster that the user must defeat during the game, depending on a guide provided before the game,
wherein the visual coordination task is a game task that moves the terminal depending on an item that the user must avoid, or an item that the user must acquire, and
wherein the processor is configured to:
adjust the weight of the visual coordination task constituting the game content within a single stage to be lower than the weight of the attention training task.

2. The apparatus of claim 1, wherein the processor is configured to:
adjust a difficulty of the game content according to at least one of a number of non-target stimuli or target stimuli, a ratio of the number of the target stimuli to a sum of the number of the non-target stimuli and the number of the target stimuli, a display time, and a length of one stage in the attention training task.

3. The apparatus of claim 2, wherein the processor is configured to:
output the target stimuli of the guide provided before the game to the terminal during the game when a performance level of the attention training task of the user is less than a predetermined performance level.

4. The apparatus of claim 1, wherein the processor is configured to:
lower a difficulty of the visual coordination task by an amount by which a difficulty of the attention training task is increased.

5. The apparatus of claim 1, wherein the processor is configured to:
additionally provide digit span data at equal or spaced time intervals within the game content in the one stage; and
determine whether input user data corresponds to the digit span data.

6. The apparatus of claim 1, wherein the processor is configured to:
normally terminate the stage at a random time point within a predetermined game time range; and
immediately terminate the stage and provide a greater reward than normal termination when a termination element is tagged during the game according to the guide provided before the game.

7. The apparatus of claim 1, wherein the processor is configured to:
in consideration of a number of consecutive failures, in which an achievement rate of in-game data of a current stage or set does not reach a predetermined value, change a difficulty of the current stage downward after the number of consecutive failures.

8. The apparatus of claim 1, wherein the processor is configured to:
output sounds corresponding to a non-target stimulus that the user must avoid during the game and a target stimulus that the user must acquire, according to an auditory guide provided before the game.

9. The apparatus of claim 1, wherein the processor is configured to:
display an achievement level for the attention training task as a response rate to a target stimulus, an omission rate to the target stimulus, a commission rate to a non-target stimulus, a non-response rate to the non-target stimulus, a response speed, and a standard deviation of the response speed; and
display an achievement level for the visual coordination task as a correct answer rate based on whether an item to be avoided is avoided and whether an item to be acquired is acquired.

10. A game providing method performed by a processor of an ADHD treatment game providing apparatus that provides tasks based on a working memory and attention, the method comprising:
acquiring in-game data during gameplay of a user in a state of being connected to a terminal providing game content,
acquiring motion data of the user during thegameplay in a state of being connected to the terminal, and
adjusting a weight of a visual coordination task and a weight of an attention training task, which constitute the game content,
wherein the attention training task is a game task that determines a monster that the user must avoid during a game, and a monster that the user must defeat during a game, depending on a guide provided before a game,
wherein the visual coordination task is a game task that moves the terminal depending on an item that the user must avoid, or an item that the user must acquire, and
wherein the adjusting of the weight includes:
adjusting the weight of the visual coordination task constituting the game content within a single stage to be lower than the weight of the attention training task.
